(19) Europäisches Patentamt
European Patent Office
Office européen des brevets

(11) Numéro de publication : **0 176 444 B1**

(12) **FASCICULE DE BREVET EUROPÉEN**

(45) Date de publication du fascicule du brevet : **22.02.89**

(51) Int. Cl.⁴ : **C 07 D249/08**, C 07 C119/14, C 07 C119/20, A 61 K 31/41

(21) Numéro de dépôt : **85401844.7**

(22) Date de dépôt : **23.09.85**

(54) **Nouveau procédé de préparation de dérivés du 4 H-1,2-4-triazole, nouveaux triazoles ainsi obtenus, leur application comme médicaments et les compositions pharmaceutiques les renfermant.**

(30) Priorité : **24.09.84 FR 8414598**

(43) Date de publication de la demande : **02.04.86 Bulletin 86/14**

(45) Mention de la délivrance du brevet : **22.02.89 Bulletin 89/08**

(84) Etats contractants désignés : **AT BE CH DE FR GB IT LI LU NL SE**

(56) Documents cités :
**FR--A-- 2 244 462**
**FR--A-- 2 269 938**
**FR--A-- 2 512 022**
**FR--A-- 2 539 127**
**GB--A-- 1 510 647**
**GB--A-- 2 082 581**
**US--A-- 2 884 424**
**DIE MAKROMOLEKULARE CHEMIE, vol. 130, 29 décembre 1969, pages 103-144, Hüthig & Wepf Verlag, Basel, CH; H.E. KÜNZEL et al.: "Über aromatische Polyamide mit heterocyclischen Ringsystemen"**
**RECHERCHE ON-LINE "DARC", version 85-01, Télésystèmes-Questel, Paris, FR; "Sur les benzenecarboximidoyl chlorides, substitués ou non dans les noyaux benzéniques"**
**CHEMICAL ABSTRACTS, vol. 66, no. 21, 22 mai 1967, page 8891, no. 94962c, Columbus, Ohio, US; R. FUSCO et al.: "C-benzoylation of 1-substituted 1,3,4-triazoles", & GAZZ. CHIM. ITAL. 96 (8-9), 1084-93 (1966)**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Clemence, Francois**
**2, rue Turgot**
**F-75009 Paris (FR)**
Inventeur : **Maushart, Claudine**
**13, allée Théophile Binet**
**F-93340 Le Raincy (FR)**
Inventeur : **Mackiewicz, Philippe**
**71 bis, avenue Turgot**
**F-93190 Livry Gargan (FR)**
Inventeur : **Delevallee, Françoise**
**48-50, avenue de LA Dame Blanche**
**F-94120 Fontenay Sous Bois (FR)**

(74) Mandataire : **Bourgouin, André et al**
**Département des Brevets ROUSSEL UCLAF B.P. no 9**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 176 444 B1

## Description

La présente invention concerne un nouveau procédé de préparation de dérivés du 4H-1,2,4-triazole, les nouveaux triazoles ainsi obtenus, leur application comme médicaments et les compositions pharmaceutiques les renfermant.

Des procédés de préparation de triazoles sont déjà décrits notamment dans le brevet anglais 1510647, le brevet US 2884 424 et dans Die Makromolekulare Chemie, vol. 130, 29 Décembre 1969, page 103-144 mais aucun de ces documents ne décrit la préparation des triazoles de la demande.

Par ailleurs, les brevets français 2512022, anglais 2082581, français 2244462 ou 2269938 décrivent des triazoles comme médicaments mais ces triazoles portent un substituant en position 3 qui est différent des substituants portés dans cette position par les nouveaux triazoles de la demande.

La présente invention a pour objet un procédé de préparation des composés de formule ($I_A$) :

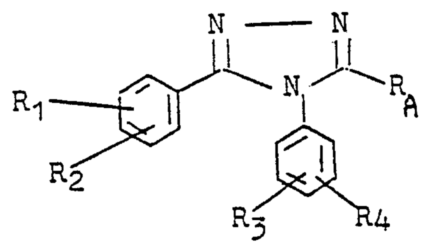

($I_A$)

dans laquelle $R_1$, $R_2$, $R_3$ et $R_4$, identiques ou différents, en position quelconque sur les noyaux benzéniques, représentent un atome d'hydrogène, un radical hydroxyle, un radical alcoyle renfermant de 1 à 4 atomes de carbone, un radical $Oalc_1$, $alc_1$ représentant un radical alcoyle renfermant de 1 à 4 atomes de carbone, un radical $NH_2$, $NHalc_2$, $alc_2$ représentant un radical alcoyle renfermant de 1 à 4 atomes de carbone ; un radical

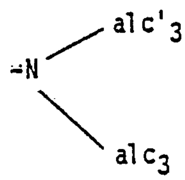

$NH_2$, $NHalc_2$, $alc_2$ représentent un radical alcoyle renfermant de 1 à 4 atomes de carbone ; un radical

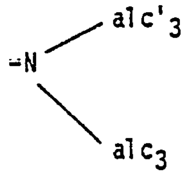

$alc_3$ et $alc'_3$, identiques ou différents représentant un radical alcoyle renfermant de 1 à 4 atomes de carbone, un atome d'halogène, un radical $CF_3$ ou un radical $NO_2$, ou $R_1$ et $R_2$ et/ou $R_3$ et $R_4$ forment ensemble un radical méthylène dioxy, $R_A$ représente un radical —CHOH—$alc_4$, ou un radical

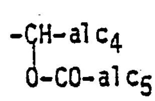

$alc_4$ et $alc_5$ identiques ou différents, représentant un radical alcoyle renfermant de 1 à 4 atomes de carbone, sous leurs formes racémiques ou optiquement actives, ainsi que de leurs sels d'addition avec les acides, caractérisé en ce que l'on soumet un composé de formule (II) :

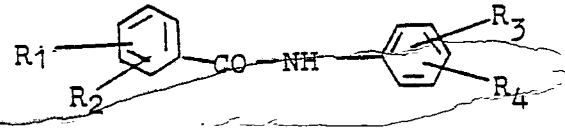

(II)

dans laquelle $R_1$, $R_2$, $R_3$ et $R_4$ ont les significations déjà indiquées, à l'action d'un agent de chloruration pour obtenir un chlorure d'imidoyle de formule III :

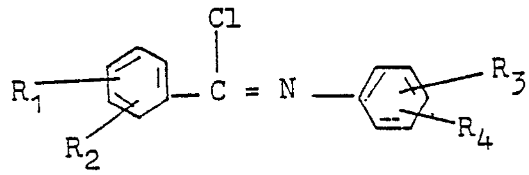

(III)

que l'on soumet à l'action d'un hydrazide de formule IV :

$$H_2N—NH—CO—Z \qquad (IV)$$

sous forme racémique ou optiquement active, dans laquelle Z est un groupe

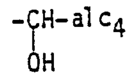

$alc_4$ étant un radical alcoyle renfermant de 1 à 4 atomes de carbone, pour obtenir un composé de formule V, sous forme racémique ou optiquement active :

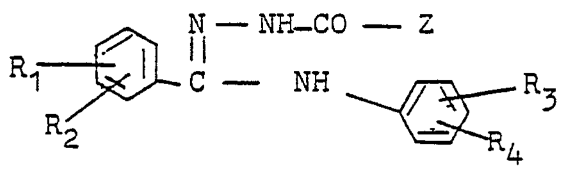

(V)

que l'on cyclise par chauffage pour obtenir un composé de formule (I), sous forme racémique ou optiquement active, dans laquelle R est un groupe

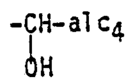

que si désiré, l'on estérifie pour obtenir un produit de formule (I) dans laquelle R est un groupe

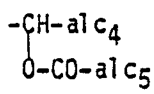

produits de formule (I), que l'on soumet, si désiré, à l'action d'un acide pour en former le sel.

Lorsque $R_1$, $R_2$, $R_3$ et $R_4$ représentent un radical alcoyle, il s'agit de préférence du radical méthyle, éthyle, n-propyle, isopropyle ou n-butyle.

$Alc_1$, $alc_2$, $alc_3$, $alc'_3$, $alc_4$ et $alc_5$ représentent de préférence un radical méthyle, éthyle, n-propyle, isopropyle ou n-butyle.

Lorsque $R_1$, $R_2$, $R_3$ et $R_4$ représentent un atome d'halogène, il s'agit d'un atome de fluor, de chlore, de brome ou d'iode et de préférence de chlore ou de brome.

La présente invention a plus particulièrement pour objet le procédé de préparation décrit ci-dessus, caractérisé en ce que l'on utilise au départ un composé de formule (II) dans laquelle un des deux substituants $R_1$ et $R_2$ représente un atome d'hydrogène et l'autre substituant représente soit un groupement méthoxy en para, soit un groupement $NO_2$ ou diméthylamino en para et dans laquelle un des deux substituants $R_3$ ou $R_4$ représente un atome d'hydrogène et l'autre substituant représente soit un groupement méthoxy en para, soit un groupement $NO_2$ ou diméthylamino en para et un hydrazide de formule IV dans laquelle Z représente un radical $—CHOH—alc_4$, $alc_4$ représentant un radical alcoyle renfermant de 1 à 4 atomes de carbone, sous forme racémique ou optiquement active, et en ce que dans le cas où $R_1$ et $R_2$ et/ou $R_3$ ou $R_4$ représentent un groupement $NO_2$ en para, l'on transforme les composés correspondants en composés dans lesquels $R_1$ ou $R_2$ et/ou $R_3$ ou $R_4$ représentent un groupement diméthylamino en para.

Il va de soi que les termes « racémiques ou optiquement actifs » ne peuvent désigner que les composés qui comportent au moins un carbone asymétrique.

La présente invention concerne aussi les composés de formule (IA) :

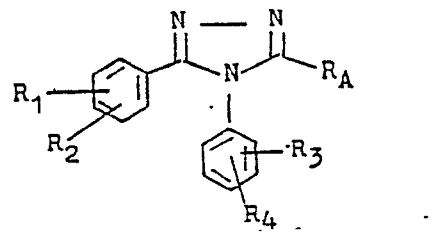

($I_A$)

dans laquelle $R_1$, $R_2$, $R_3$ et $R_4$, identiques ou différents, en position quelconque sur les noyaux benzéniques, représentent un atome d'hydrogène, un radical hydroxyle, un radical alcoyle renfermant de 1 à 4 atomes de carbone, un radical $Oalc_1$, $alc_1$ représentant un radical alcoyle renfermant de 1 à 4 atomes de carbone, un radical $NH_2$, $NHalc_2$, $alc_2$ représentant un radical alcoyle renfermant de 1 à 4 atomes de carbone, un radical

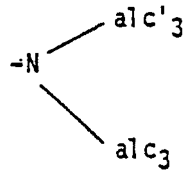

$alc_3$ et $alc'_3$, identiques ou différents, représentant un radical alcoyle renfermant de 1 à 4 atomes de carbone, un atome d'halogène, un radical $CF_3$ ou un radical $NO_2$, ou $R_1$ et $R_2$ et/ou $R_3$ et $R_4$ forment ensemble un radical méthylènedioxy et dans laquelle $R_A$ représente un radical —CHOH—$alc_4$, ou un radical

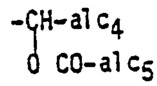

$alc_4$ et $alc_5$ identiques ou différents représentant un radical alcoyle renfermant de 1 à 4 atomes de carbone, sous leurs formes racémiques ou optiquement actives, ainsi que leurs sels d'addition avec les acides.

Parmi les sels d'addition avec les acides, on peut citer ceux formés avec les acides minéraux tels que les acides chlorhydrique, bromhydrique, sulfurique ou phosphorique, ou les acides organiques tels que les acides acétique, propionique, maléique ou hémisuccinique.

L'invention concerne notamment les composés de formule IA pour lesquels un des deux substituants $R_1$ ou $R_2$ représente un atome d'hydrogène et l'autre substituant représente soit un groupement méthoxy en para, soit un groupement diméthylamino en para et pour lesquels un des deux substituants $R_3$ ou $R_4$ représente un atome d'hydrogène et l'autre substituant représente soit un groupement méthoxy en para, soit un groupement diméthylamino en para, sous leurs formes racémiques ou optiquement actives, ainsi que leurs sels d'addition avec les acides et tout particulièrement le 5-/4-(diméthylamino) phényl/ 4-(4-méthoxy phényl) α-méthyl 4H-1,2,4-triazole-3-méthanol, sous forme racémique ou optiquement active et ses sels d'addition avec les acides.

Des triazoles de formule (I) correspondant aux triazoles de formule $I_A$ dans laquelle $R_A$ correspond à un radical alcoyle R renfermant de 1 à 4 atomes de carbone sont décrits dans le brevet belge n° 890035 et sont préparés dans ce brevet notamment par le procédé suivant :

le composé de formule III' :

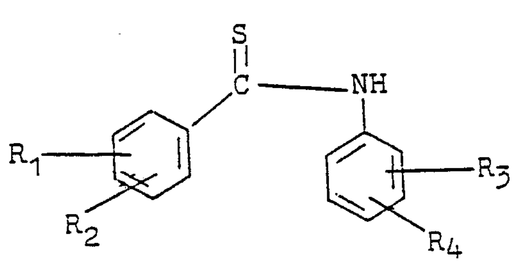

(III')

est soumis à l'action de l'hydrazine pour obtenir un composé de formule :

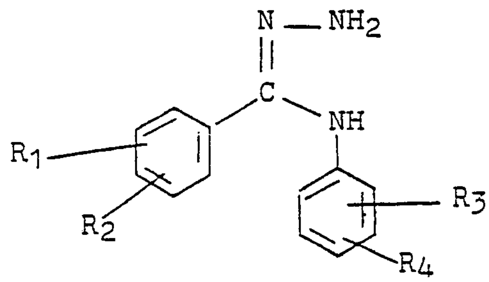

(IV')

que l'on soumet à l'action d'un acide ou d'un dérivé fonctionnel d'acide $XCO_2H$ dans lequel X représente notamment un radical alcoyle renfermant de 1 à 4 atomes de carbone, pour obtenir le composé de formule V' :

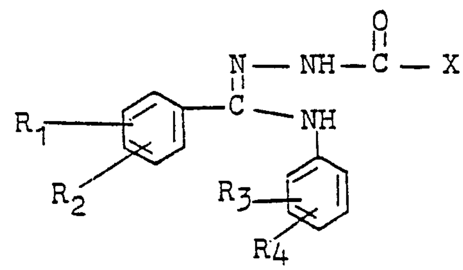

(V')

que l'on cyclise par chauffage, pour obtenir le composé de formule (I), dans laquelle $R_1$, $R_2$, $R_3$ et $R_4$ sont définis comme précédemment et R représente un radical alcoyle renfermant de 1 à 4 atomes de carbone.

Dans le procédé décrit précédemment, l'amidrazone IV' intermédiaire obtenue est instable et des impuretés, notamment des tétrazines, se forment lors de sa synthèse. La formation de ces produits de dégradation rend difficile la purification de l'amidrazone et de l'acylamidrazone V' formée ensuite.

Dans le nouveau procédé de l'invention, il n'y a pas d'intermédiaire instable, l'acylamidrazone V' est obtenue facilement ; elle est stable, exempte d'impuretés et donc facilement purifiable.

Dans un mode de réalisation préférée du procédé de l'invention :

Le réactif chloré permettant d'obtenir le chlorure d'imidoyle III est avantageusement le chlorure de thionyle ou le phosgène. Le pentachlorure de phosphore ou l'oxychlorure de phosphore peuvent aussi être utilisés.

La réaction permettant d'obtenir le chlorure d'imidoyle III s'effectue éventuellement sans solvant, simplement au reflux du réactif de chloruration utilisé.

Si la réaction s'effectue en présence d'un solvant, celui-ci est par exemple, le toluène, le benzène ou le dichlorométhane.

Dans le cas où $R_1$, $R_2$, $R_3$ ou $R_4$ représente une fonction sensible, telle que par exemple la fonction

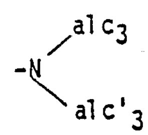

le réactif de chloruration est de préférence le phosgène et la réaction est réalisée à basse température, de préférence entre — 30 °C et — 60 °C. Par ailleurs, dans ce cas particulier, la réaction peut s'effectuer en présence d'une base tertiaire, telle que la pyridine ou la triéthylamine qui permet d'éliminer l'acide chlorhydrique formé.

La condensation entre le chlorure d'imidoyle III et l'hydrazide IV s'effectue dans un solvant tel que le toluène, le benzène et la réaction s'effectue éventuellement au reflux de ce solvant.

La réaction d'estérification pour obtenir un produit de formule (I) dans laquelle $R_A$=

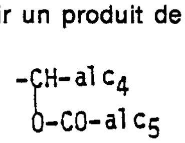

s'effectue dans les conditions usuelles.

Il va de soi que l'on peut apporter aux substituants $R_1$, $R_2$, $R_3$ et $R_4$ une fois un composé de formule (I) préparé, des variations évidentes pour un chimiste. On peut, par exemple, éthérifier ou estérifier un hydroxyle, cliver un groupement O-alcoyle pour obtenir un groupement hydroxyle, transformer le groupement $NO_2$ en un groupement $NH_2$ ou en un groupement N $(alcoyle)_2$, substituer un dérivé fluoré par une amine.

Les composés de formule $I_A$ tels que définis ci-dessus, ainsi que leurs sels d'addition avec les acides, présentent d'intéressantes propriétés pharmacologiques. Ils présentent, en particulier, une très bonne activité analgésique. En outre, le 5-/4-(diméthylamino) phényl/ 4-(4-méthoxyphényl) α-méthyl 4H-1,2,4-triazole 3-méthanol possède une certaine activité anti-inflammatoire dans les modèles d'inflammation chronique.

Ces propriétés justifient leur application en thérapeutique et l'invention a également pour objet à titre de médicaments, les produits tels que définis par la formule $I_A$ ci-dessus, ainsi que les sels d'addition avec les acides pharmaceutiquement acceptables desdits produits de formule $I_A$.

La présente invention concerne notamment, à titre de médicament, les composés de formule $I_A$ pour lesquels un des deux substituants $R_1$ ou $R_2$ représente un atome d'hydrogène, et l'autre substituant représente soit un groupement méthoxy en para, soit un groupement diméthylamino en para et pour lesquels un des deux substituants $R_3$ ou $R_4$ représente un atome d'hydrogène, et l'autre substituant représente soit un groupement méthoxy en para, soit un groupement diméthylamino en para sous leurs formes racémiques ou optiquement actives, ainsi que leurs sels d'addition avec les acides pharmaceutiquement acceptables.

La présente invention a tout particulièrement pour objet, à titre de médicaments, le 5-/4-(diméthylamino) phényl/ 4-(4-méthoxy phényl) α-méthyl 4H-1,2,4-triazole-3-méthanol et ses sels d'addition avec les acides pharmaceutiquement acceptables.

Les médicaments, objet de l'invention, peuvent être utilisés dans le traitement des algies musculaires, articulaires ou nerveuses, des affections rhumatismales, les douleurs dentaires, des zonas et des migraines, et aussi à titre de traitement complémentaire dans les états infectieux et fébriles.

Le 5-/4-(diméthylamino) phényl/ 4-(4-méthoxy phényl) α-méthyl 4H-1,2,4-triazole-3-méthanol peut, quant à lui, être préconisé dans le traitement des maladies inflammatoires dégénératives telles que l'ostéoarthrose, les collagénoses diverses (tendinites), des maladies rhumatismales, la polyarthrite rhumatoïde, spondylarthrite ankylosante), ainsi que dans le traitement d'autres maladies de nature auto immune telles que le lupus érythémateux disséminé, les glomérulonéphrites, la sclérose en plaques.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

Les composés de formule II utilisés comme produits de départ du procédé de l'invention sont préparés comme indiqué dans le brevet belge N° 890 035. (Dans ce brevet, les produits de formule II ne sont pas isolés, car ils réagissent directement avec le pentasulfure de phosphore pour donner les produits de formule III'.)

Les composés de formule IV sont préparés classiquement en faisant réagir sur l'hydrate d'hydrazine, un produit de formule :

$$CH_3O-\underset{\underset{O}{\|}}{C}-R \quad \text{ou} \quad C_2H_5O\underset{\underset{O}{\|}}{C}-R$$

R ayant la signification donnée pour les produits de formule I.

Les exemples donnés ci-dessous illustrent l'invention sans toutefois la limiter.

Exemple de référence n° 1 : 3-éthyl 4-(4-méthoxyphenyl) 5-(4-nitrophényl) 4H-1,2,4-triazole

Stade A : chlorure de N-(4-méthoxy phényl) 4-nitrobenzène carboximidoyle

On chauffe au reflux pendant 6 heures, 1,36 g de N-(4-méthoxy phényl) 4-nitrobenzamide (préparation donnée ci-dessous) et 5 $cm^3$ de chlorure de thionyle, ajoute ensuite 30 $cm^3$ de benzène, élimine le benzène et le chlorure de thionyle sous pression réduite et obtient le produit attendu fondant à 120 °C-122 °C.

Préparation du N-(4-méthoxy phényl) 4-nitrobenzamide

On ajoute par petites fractions 12,3 g de para-anisidine dans une suspension contenant 18,6 g de chlorure d'acide 4-nitrobenzoïque dans 100 $cm^3$ de pyridine, chauffe 2 heures au reflux. On revient à température ambiante, verse la solution dans 500 $cm^3$ d'eau glacée, essore le précipité formé, lave à l'eau, sèche sous pression réduite à 80 °C et obtient 20 g de produit attendu fondant à 200 °C.

Stade B : 2-(1-oxopropyl) hydrazide de l'acide N-(4-méthoxy phényl) 4-nitrobenzène carboximique

On ajoute 2,8 $cm^3$ de triéthylamine à une solution renfermant 1,76 g d'hydrazide de l'acide propionique (préparation donnée ci-dessous) dans 80 $cm^3$ de toluène. On ajoute ensuite en une fois 2,87 g de produit obtenu au stade A, agite 15 minutes, essore, sèche, reprend le résidu obtenu dans 100 $cm^3$ d'eau, extrait au chlorure de méthylène, sèche, concentre à sec et obtient 2,2 g de produit attendu fondant à 158 °C.

Préparation de l'hydrazide de l'acide propionique

On porte 18 heures au reflux 38,83 $cm^3$ d'hydrate d'hydrazine et 52,42 g de propionate d'éthyle. On obtient le produit attendu $E/_{6mmHg}$ = 100 °C.

Stade C : 3-éthyl 4-(4-méthoxyphényl) 5-(4-nitrophényl) 4H-1,2,4-triazole

On porte 2 heures au reflux, 4,38 g de produit obtenu au stade B dans 60 $cm^3$ de toluène, évapore le toluène, reprend le résidu à l'éther isopropylique et recristallise le produit obtenu dans un mélange d'éther isopropylique et d'isopropanol (2-1). On obtient le produit attendu fondant à 174 °C.

Exemple de référence n° 2 : 3-éthyl 4-(4-méthoxy phényl) 5-(4-nitro 4-méthylphényl) 4H-1,2,4-triazole

Stade A : chlorure de N-(4-méthoxyphényl) 4-nitro 3-méthyl benzène carboximidoyle

On chauffe 20 heures au reflux 14 g de N-(4-méthoxyphényl) 4-nitro 3-méthyl benzamide (préparation donnée ci-dessous) en suspension dans 70 $cm^3$ de chlorure de thionyle.

On élimine le solvant par entraînement au benzène, sèche sous pression réduite à température ambiante et obtient 15 g de produit fondant vers 80 °C.

Préparation du N-(4-méthoxyphényl) 4-nitro 3-méthylbenzamide

On opère comme pour la préparation de la N-(4-méthoxyphényl) 4-nitrobenzamide (voir stade A, exemple de référence n° 1) à partir de 15,5 g de chlorure de 4-nitrométatoluyle et de 9,56 g de para-anisidine en présence de pyridine. On obtient 16,7 g de produit attendu.

Stade B : 3-éthyl 4-(4-méthoxyphényl) 5-(4-nitro 3-méthylphényl) 4H-1,2,4-triazole

On introduit 1,4 $cm^3$ de triéthylamine, puis 3 g de produit obtenu au stade A dans une solution renfermant 1,76 g d'hydrazide de l'acide propionique dans 160 $cm^3$ de toluène, celle-ci ayant été portée au reflux puis refroidie. On porte 1 heure au reflux, élimine le toluène sous pression réduire, reprend le résidu par 50 $cm^3$ de chloroforme et 50 $cm^3$ d'eau. On extrait la phase aqueuse au chloroforme, sèche, concentre, et obtient une huile que l'on purifie par chromatographie sur silice (éluant acétate d'éthyle-cyclohexane (8-2)). On obtient 3 g d'une huile présentant un Rf. de 0,6 en chromatographie sur couche mince (éluant : chloroforme méthanol 9-1).

Exemple de référence n° 3 : N,N-diméthyl 4-/5-éthyl 4-(4-méthoxyphényl) 4H-1,2,4-triazol-3-yl/2-méthyl benzénamine

On refroidit à + 5 °C, 2,6 g de produit obtenu à l'exemple de référence n° 2, 13 $cm^3$ d'aldéhyde formique et 5,2 g de zinc en poudre, puis introduit lentement 13 $cm^3$ d'acide acétique pur. On laisse remonter à température ambiante, agite 20 heures, essore et lave le zinc par 25 $cm^3$ du mélange acide acétique-eau, 100 $cm^3$ de chlorure de méthylène, 50 $cm^3$ d'eau.

On décante le filtrat, extrait la phase aqueuse au chlorure de méthylène, lave la phase organique à l'eau, la sèche, la concentre.

On obtient 3 g d'une huile qui est reprise par 20 $cm^3$ d'éther isopropylique puis refroidie à — 50 °C. On revient à température ambiante, essore et sèche sous pression réduite à 60 °C.

On obtient 1,85 g de produit que l'on recristallise deux fois dans un mélange d'acétate d'éthyle et d'éther isopropylique (4-6).

On obtient 1,2 g de produit attendu fondant à 131 °C.

Exemple de référence n° 4 : 3-éthyl 4-(4-méthoxyphényl) 5-/4-(NN-diméthylamino) phényl/4H-1,2,4-triazole

On introduit sous argon et sous agitation 1 kg de 4-diméthylamino 4-méthoxy benzanilide (préparation donnée ci-dessous) 8 litres de dichlororméthane et 1 130 $cm^3$ de triéthylamine, refroidit à — 40 °C ± 1 °C et introduit à cette température 2,6 litres de solution de phosgène dans le toluène, agite, réchauffe à 20 °C en 2 heures.

On introduit la solution ainsi obtenue dans 40 litres de toluène chauffé précédemment au reflux et ajoute simultanément une deuxième solution contenant 489 g d'hydrazide de l'acide propionique dans 489 $cm^3$ de dichlorométhane tout en maintenant la température du milieu réactionnel ⩾ 90 °C.

On distille en continu tout au long de l'introduction des deux solutions. Le volume ainsi distillé en fin d'introduction est d'environ 30 litres et on arrête le chauffage lorsque la température du milieu réactionnel est ⩾ 100 °C.

On refroidit à 20 °C et lave successivement à l'eau, à l'eau contenant de la lessive de soude (4-1) et à l'eau.

On extrait au dichlorométhane, sèche, ajoute 100 g de charbon actif, agite, essore, lave au dichlorométhane, concentre par distillation sous pression atmosphérique jusqu'à un volume de 2 litres.

On refroidit à 20 °C et maintient 16 heures sous agitation.

On essore, lave au toluène, sèche à 60 °C et obtient 984 g de produit brut.

On récupère également 61,5 g de produit brut à partir des liqueurs mères de cristallisation toluéniques.

Purification

On chauffe au reflux, sous agitation et sous azote, 1 045 g de produit brut dans 4 182 $cm^3$ de toluène, ajoute en 15 minutes 104,5 g de charbon actif, maintient sous agitation au reflux pendant 15 minutes, essore à chaud, lave au toluène bouillant puis refroidit à 20 °C, agite une nuit à 20 °C, essore, lave au toluène, sèche à 60 °C et obtient 862,6 g de produit attendu fondant à 167 °C.

Préparation du 4-diméthylamino 4-méthoxybenzanilide

On ajoute 201 g de para anisidine dans une suspension de 30 g de chlorure d'acide 4-diméthylamino benzoïque dans 1 200 $cm^3$ de pyridine, agite jusqu'à dissolution puis chauffe 3 heures au reflux. On refroidit à température ambiante, verse le milieu réactionnel dans 6 litres d'eau glacée, essore le précipité, lave à l'eau, sèche sous pression réduite et obtient 423 g de produit attendu. F = 173 °C.

Exemple 1 : 5-/4-nitrophényl/4-(4-méthoxyphényl)méthyl 4H-1,2,4-triazol-3-méthanol (forme L)

On ajoute 3 $cm^3$ de triéthylamine à température ambiante dans une solution renfermant 2 équivalents d'hydrazide de l'acide lactique (préparation donnée ci-dessous) dans 60 $cm^3$ de toluène. On ajoute ensuite, en 2 fois, 4,3 g de chlorure de N-(4-méthoxyphényl) 4-nitrobenzène carboximidoyle (stade A de l'exemple de référence n° 1) et agite 4 heures à température ambiante.

On porte ensuite 4 heures au reflux pour obtenir la cyclisation élimine le toluène sous pression réduite, lave à l'eau, essore, sèche et obtient 4,34 g de produit brut que l'on purifie par chromatographie sous pression (éluant : chloroforme — acétate d'éthyle — méthanol : 47-50-3) et obtient 3 g de produit attendu fondant à 140 °C.

Préparation de l'hydrazide de l'acide β lactique

On introduit lentement à 50 °C, 9,7 $cm^3$ de lactate d'éthyle (forme L) dans 4,1 $cm^3$ d'hydrate d'hydrazine, porte au reflux 4 heures, évapore sous pression réduite et récupère une huile incolore utilisée telle quelle pour la préparation du produit de l'exemple 1.

Exemple 2 : 5-/4-(diméthylamino)-phényl/4-(4-méthoxyphényl)α-méthyl 4H-1,2,4-triazol-3-méthanol (forme L)

On opère, comme indiqué à l'exemple de référence n° 3, à partir de 3 g de produit de l'exemple 10, 6 g de zinc en poudre, 15 $cm^3$ d'aldéhyde formique et 15 $cm^3$ d'acide acétique. On obtient 1,12 g de produit attendu fondant à 145 °C.

Exemple 3 : Acétate de 5-/4-(diméthylamino) phényl/4-(4-méthoxy-phényl)α-méthyl 4H-1,2,4-triazol 3-méthanol (forme L)

On ajoute 0,47 g d'hydrure de sodium à une suspension contenant 3,3 g du produit préparé à l'exemple 2 dans 33 $cm^3$ de tétrahydrofuranne. On agite 30 minutes, ajoute 0,92 g de chlorure d'acétyle et

maintient 16 heures l'agitation sous atmosphère inerte. On concentre à sec, reprend l'extrait dans 30 $cm^3$ d'eau, agite 30 minutes, essore le précipité, le lave à l'eau et le sèche. Après purification par chromatographie sous pression (éluant : acétate d'éthyle-chloroforme 8-2), on obtient 2,8 g de produit attendu que l'on recristallise dans le toluène. F = 208 °C. $[\alpha]_D = -16° \pm 1°$ (c = 1 % chloroforme).

Analyse : $C_{21}H_{24}N_4O_3$ : 380,450
Calculé : C 66,30 H 6,36 N 14,73 %
Trouvé : C 66,0 H 6,3 N 14,5 %

Exemple 4 : 5-/4-(diméthylamino)phényl/4-(4-méthoxyphényl)α-méthyl 4H-1,2,4-triazol 3-méthanol (forme DL)

On ajoute 2,36 g de triéthylamine dans une suspension comprenant 5 g de 4-diméthylamino 4-méthoxy benzanilide (préparé comme à l'exemple de référence n° 4) dans 50 $cm^3$ de chlorure de méthylène, refroidit à — 40 °C, ajoute en 20 minutes sous atmosphère inerte une solution de 5,75 $cm^3$ de phogène dans le toluène et réchauffe à 20 °C en 1 heure et 30 minutes. On introduit la solution ainsi obtenue dans 200 $cm^3$ de toluène chauffé au reflux, et ajoute simultanément une solution contenant 2,9 g d'hydrazide de l'acide lactique (forme DL) dans 6 $cm^3$ de diméthylformamide. On élimine par distillation une partie des solvants, chauffe pendant 30 minutes jusqu'à 107-110 °C, refroidit le milieu réactionnel, le lave à l'eau puis avec une solution aqueuse de soude N et à l'eau. On extrait au chlorure de méthylène, concentre à sec, obtient 7,1 g de produit brut que l'on recristallise dans le mélange acétylacétate d'éthyle-éther isopropylique (3-6) puis dans l'acétate d'éthyle pur. F = 170 °C.

Analyse : $C_{19}H_{22}N_4O_2$ : 338,413
Calculé : C 67,43 H 6,55 N 16,55 %
Trouvé : C 67,2 H 6,8 N 16,3 %

Préparation de l'hydrazide de l'acide lactique (forme DL)

On chauffe 4 heures 30 minutes à 100/110 °C, 50 g de DL-lactate de méthyle et 24,2 g d'hydrate d'hydrazide. On élimine l'eau par entraînement au xylène, et obtient le produit attendu sous forme d'huile qu'on utilise telle quelle pour la préparation du produit de l'exemple 4.

Exemple 5 : Acétate de 5-/4-(diméthylamino) phényl/4-(4-méthoxy-phényl)α-méthyl 4H-1,2,4-triazol 3-méthanol (forme DL)

On opère comme à l'exemple 3 à partir de 2,4 g du produit préparé à l'exemple 4, 0,336 g d'hydrure de sodium et 0,660 g de chlorure d'acétyle. On obtient 2,24 g de produit attendu que l'on recristallise dans le toluène. F = 190 °C.

Analyse : $C_{21}H_{24}N_4O_3$ : 380,450
Calculé : C 66,3 H 6,36 N 14,73 %
Trouvé : C 66,4 H 6,5 N 14,8 %

Exemple 6 : Compositions pharmaceutiques

On a préparé des comprimés répondant à la formule suivante :

| | |
|---|---|
| Produit de l'exemple 2 | 50 mg |
| Excipient q.s pour un comprimé terminé à | 350 mg |

(détail de l'excipient : lactose, talc, amidon, stéarate de magnésium).

Exemple 7 : Etude pharmacologique du produit de l'exemple 2

Activité analgésique

Le test employé est basé sur le fait signalé par R. KOSTER et Coll., (Fed. Proc. 1959, 18412) selon lequel l'injection intrapéritonéale d'acide acétique provoque, chez la souris, des mouvements répétés d'étirement et de torsion pouvant persister pendant plus de six heures. Les analgésiques préviennent ou diminuent ce syndrome qui peut être considéré comme l'extériorisation d'une douleur abdominale diffuse. On utilise une solution d'acide acétique à 1 % dans l'eau. La dose déclenchant le syndrome est dans ces conditions de 0,01 $cm^3$/g, soit 100 mg/kg d'acide acétique.

Le produit étudié est administré par voie buccale une demi-heure avant l'injection d'acide acétique, les souris étant à jeun depuis la veille de l'expérience.

Les étirements sont observés et comptés pour chaque souris pendant une période d'observation de quinze minutes commençant aussitôt après l'injection d'acide acétique.

Les résultats sont exprimés au moyen de la $DA_{50}$, c'est-à-dire la dose qui permet d'obtenir une diminution de 50 % du nombre des étirements par rapport aux animaux témoins.

La $DA_{50}$ trouvée a été de 4 mg/kg.

Activité anti-inflammatoire : arthrite chronique à l'adjuvant (traitement préventif)

L'injection d'adjuvant du type Freund dans une patte postérieure provoque, chez le rat, l'apparition rapide d'une lésion inflammatoire primaire dans cette patte, puis, après un temps de latence de 13 à 15 jours, le déclenchement d'une arthrite secondaire affectant notamment l'autre patte postérieure. Le test est pratiqué sur des rats mâles âgés de 42 à 50 jours, qui reçoivent en injection intraplantaire, 0,1 ml d'adjuvant de type « Freund » (suspension dans l'huile de vaseline de 6 mg par ml de mycobacterium butyricum tués).

Les animaux reçoivent le produit étudié, par voie orale, du jour 0 (jour de l'injection de l'adjuvant) jusqu'à la veille du sacrifice, pratiqué le jour 17. Des animaux témoins arthritiques, des animaux témoins normaux ne reçoivent que le véhicule. Les critères d'appréciation de l'activité des substances étudiées sont les augmentations de volume des pattes postérieures non injectées (inflammation secondaire) par rapport au volume moyen des pattes correspondantes de témoins normaux.

On détermine la $DA_{50}$, c'est-à-dire la dose qui diminue de 50 % les augmentations de volume des pattes postérieures des animaux traités par rapport aux animaux témoins.

Le résultat obtenu a traduit une activité notable du produit de l'exemple 2 sur ce test.

**Revendications**

1. Procédé de préparation des composés de formule (IA) :

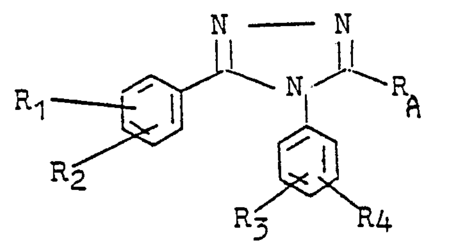

($I_A$)

dans laquelle $R_1$, $R_2$, $R_3$ et $R_4$, identiques ou différents, en position quelconque sur les noyaux benzéniques, représentent un atome d'hydrogène, un radical hydroxyle, un radical alcoyle renfermant de 1 à 4 atomes de carbone, un radical $Oalc_1$, $alc_1$ représentant un radical alcoyle renfermant de 1 à 4 atomes de carbone, un radical $NH_2$, $NHalc_2$, $alc_2$ représentant un radical alcoyle renfermant de 1 à 4 atomes de carbone ; un radical

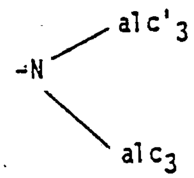

$alc_3$ et $alc'_3$, identiques ou différents, représentant un radical alcoyle renfermant de 1 à 4 atomes de carbone, un atome d'halogène, un radical $CF_3$ ou un radical $NO_2$, ou $R_1$ et $R_2$ et/ou $R_3$ et $R_4$ forment ensemble un radical méthylènedioxy, $R_A$ représente un radical —CHOH-$alc_4$, ou un radical

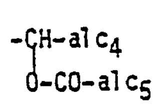

$alc_4$ et $alc_5$ identiques ou différents, représentant un radical alcoyle renfermant de 1 à 4 atomes de carbone, sous leurs formes racémiques ou optiquement actives, ainsi que de leurs sels d'addition avec les acides, caractérisé en ce que l'on soumet un composé de formule (II) :

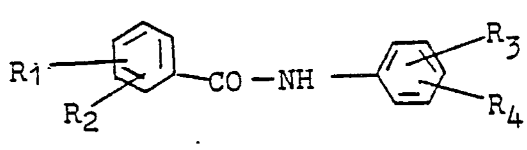

(II)

dans laquelle $R_1$, $R_2$, $R_3$ et $R_4$ ont les significations déjà indiquées, à l'action d'un agent de chloruration pour obtenir un chlorure d'imidoyle de formule III :

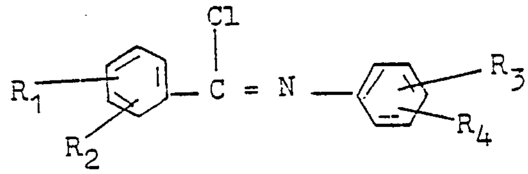

(III)

que l'on soumet à l'action d'un hydrazide de formule IV :

$$H_2N—NH—CO—Z \quad (IV)$$

sous forme racémique ou optiquement active, dans laquelle Z est un groupe

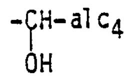

$alc_4$ étant un radical alcoyle renfermant de 1 à 4 atomes de carbone, pour obtenir un composé de formule V, sous forme racémique ou optiquement active :

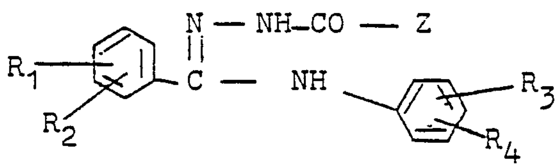

(V)

que l'on cyclise par chauffage pour obtenir un composé de formule (I), sous forme racémique ou optiquement active, dans laquelle R est un groupe

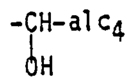

que si désiré, l'on estérifie pour obtenir un produit de formule (I) dans laquelle $R_A$ est un groupe

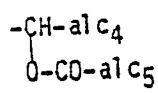

produits de formule (I), que l'on soumet, si désiré, à l'action d'un acide pour en former le sel.

2. Procédé selon la revendication 1, caractérisé en ce que l'on utilise au départ un composé de formule II dans laquelle un des deux substituants $R_1$ ou $R_2$ représente un atome d'hydrogène et l'autre substituant représente soit un groupement méthoxy en para, soit un groupement $NO_2$ ou diméthylamino en para et dans laquelle un des deux substituants $R_3$ ou $R_4$ représente un atome d'hydrogène et l'autre substituant représente soit un groupement méthoxy en para, soit un groupement $NO_2$ ou diméthyl amino en para et un hydrazide de formule IV dans laquelle Z représente un radical —CHOH-$alc_4$, $alc_4$, représentant un radical alcoyle renfermant de 1 à 4 atomes de carbone.

3. Les composés de formule (IA) :

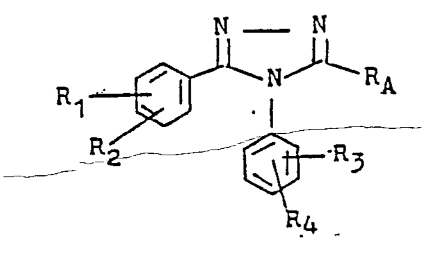

($I_A$)

dans laquelle $R_1$, $R_2$, $R_3$, $R_4$ et RA ont les significations données à la revendication 1 sous leurs formes racémiques ou optiquement actives, ainsi que leurs sels d'addition avec les acides.

4. Les composés de formule (IA) tels que définis à la revendication 3 pour lesquels un des deux substituants $R_1$ ou $R_2$ représente un atome d'hydrogène, et l'autre substituant représente soit un groupement méthoxy en para, soit un groupement diméthylamino en para et pour lesquels un des deux substituants $R_3$ ou $R_4$ représente un atome d'hydrogène, et l'autre substituant représente soit un groupement méthoxy en para, soit un groupement diméthylamino en para sous leurs formes racémiques ou optiquement actives, ainsi que leurs sels d'addition avec les acides.

5. Le 5-/4-(diméthylamino) phényl/ -(4-méthoxy phényl) 4-méthyl 4H - 1,2,4 triazole - 3 - méthanol, sous forme racémique ou optiquement active et ses sels d'addition avec les acides.

6. A titre de médicaments, les composés de formule (IA), tels que définis aux revendications 3 et 4 ainsi que leurs sels d'addition avec les acides pharmaceutiquement acceptables.

**Claims**

1. Process for the preparation of compounds of formula ($I_A$) :

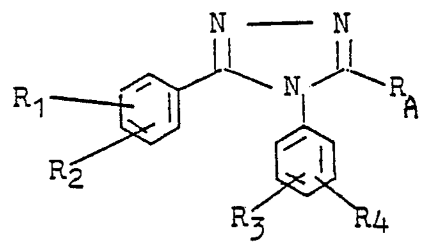

($I_A$)

in which $R_1$, $R_2$, $R_3$ and $R_4$, identical or different, in whatever position on the benzene nuclei, represent a hydrogen atom, an hydroxyl radical, an alkyl radical containing from 1 to 4 carbon atoms, an $Oalk_1$ radical, $alk_1$ representing an alkyl radical containing from 1 to 4 carbon atoms, an $NH_2$, $NHalk_2$ radical, $alk_2$ representing an alkyl radical containing from 1 to 4 carbon atoms ; a radical

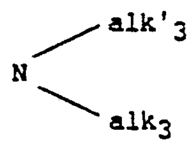

$alk_3$ and $alk'_3$, identical or different, representing an alkyl radical containing from 1 to 4 carbon atoms, a halogen atom, a $CF_3$ radical or an $NO_2$ radical, or $R_1$ and $R_2$ and/or $R_3$ and $R_4$ together form a methylenedioxy radical, $R_A$ represents a —CHOH—$alk_4$ radical, or a

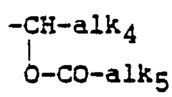

radical, $alk_4$ and $alk_5$, identical or different, representing an alkyl radical containing from 1 to 4 carbon atoms, in their racemic or optically active forms, as well as their addition salts with acids characterized in that a compound of formula (II) :

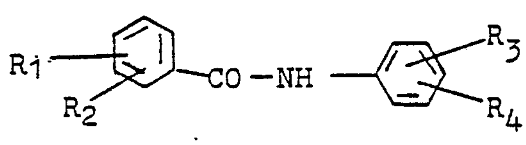

(II)

in which $R_1$, $R_2$, $R_3$ and $R_4$ have the significances already indicated, is submitted to the action of a chlorination agent so as to obtain an imidyl chloride of formula (III) :

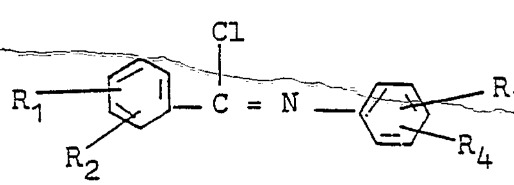

(III)

which is submitted to the action of a hydrazide of formula (IV)

$H_2N—NH—CO—Z$ (IV)

in racemic or optically active form, in which Z is a

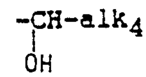

group, $alk_4$ being an alkyl radical containing from 1 to 4 carbon atoms, so as to obtain a compound of formula (V), in racemic or optically active form :

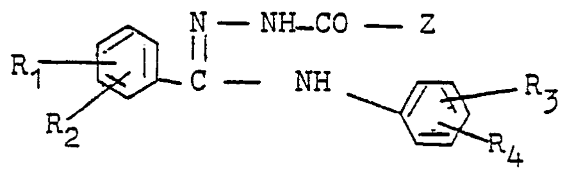

(V)

which is cyclized by heating so as to obtain a compound of formula (I), in racemic or optically active form, in which R is a

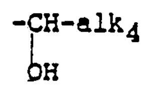

group, which if desired is esterified so as to obtain a product of formula (I) in which $R_A$ is a

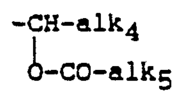

group, which products of formula (I) are submitted, if desired, to the action of an acid so as to form a salt.

2. Process according to claim 1, characterized in that at the start a compound of formula (II) is used in which one of the two substituents $R_1$ or $R_2$ represents a hydrogen atom and the other substituent represents either a methoxy group in the para position, or an $NO_2$ or dimethylamino group in the para position and in which one of the two substituents $R_3$ or $R_4$ represents a hydrogen atom and the other substituent represents either a methoxy group in the para position, or an $NO_2$ or dimethylamino group in the para position and a hydrazide of formula (IV) in which Z represents a $—CHOH\text{-}alk_4$ radical, $alk_4$ representing an alkyl radical containing from 1 to 4 carbon atoms.

3. Compounds of formula ($I_A$) :

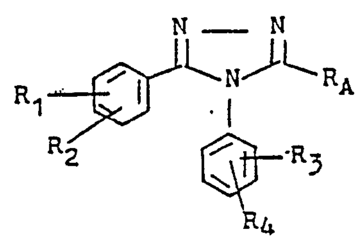

($I_A$)

in which $R_1$, $R_2$, $R_3$, $R_4$ and $R_A$ have the significances given in claim 1, in their racemic or optically active forms, as well as their addition salts with acids.

4. Compounds of formula ($I_A$) as defined in claim 3 for which one of the two substituents $R_1$ or $R_2$ represents a hydrogen atom, and the other substituent represents either a methoxy group in the para position, or a dimethylamino group in the para position and for which one of the substituents $R_3$ or $R_4$ represents a hydrogen atom, and the other substituent represents either a methoxy group in the para position, or a dimethylamino group in the para position, in their racemic or optically active forms, as well as their addition salts with acids.

5. 5-[4-(dimethylamino)phenyl]-(4-methoxyphenyl)-4-methyl-4H-1,2,4-triazole-3-methanol, in racemic or optically active form and its addition salts with acids.

6. As medicaments, the compounds of formula ($I_A$), as defined in claims 3 and 4, as well as their addition salts with pharmaceutically acceptable acids.

**Patentansprüche**

1. Verfahren zur Herstellung der Verbindungen der Formel ($I_A$)

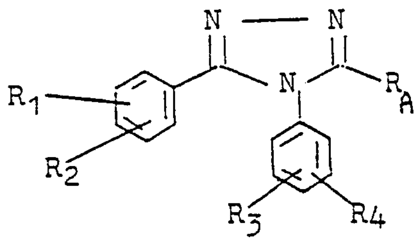

($I_A$)

worin $R_1$, $R_2$, $R_3$ und $R_4$, die gleich oder voneinander verschieden sind, in beliebiger Stellung an den Benzolringen ein Wasserstoffatom, einen Hydroxylrest, einen Alkylrest mit 1 bis 4 Kohlenstoffatomen, einen Rest $Oalc_1$, worin $alc_1$ einen Alkylrest mit 1 bis 4 Kohlenstoffatomen darstellt, einen Rest $NH_2$, $NHalc_2$, worin $alc_2$ einen Alkylrest mit 1 bis 4 Kohlenstoffatomen darstellt ; einen Rest

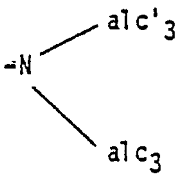

worin $alc_3$ und $alc'_3$, die gleich oder voneinander verschieden sind, einen Alkylrest mit 1 bis 4 Kohlenstoffatomen darstellen, ein Halogenatom, einen $CF_3$-Rest oder einen $NO_2$-Rest bedeuten oder $R_1$ und $R_2$ und/oder $R_3$ und $R_4$ gemeinsam einen Methylendioxy-Rest bilden, $R_A$ einen Rest —CHOH-$alc_4$ oder einen Rest

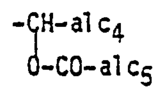

darstellt, worin $alc_4$ und $alc_5$, die gleich oder voneinander verschieden sind, einen Alkylrest mit 1 bis 4 Kohlenstoffatomen bedeuten, in ihren racemischen oder optisch aktiven Formen sowie von deren Additionssalzen mit Säuren, dadurch gekennzeichnet, daß man eine Verbindung der Formel (II)

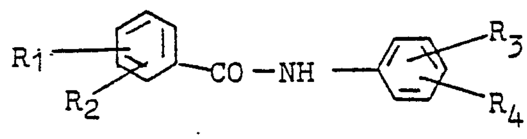

(II)

worin $R_1$, $R_2$, $R_3$ und $R_4$ die angegebene Bedeutung besitzen, der Einwirkung eines Chlorierungsmittels aussetzt, um ein Imidoylchlorid der Formel III

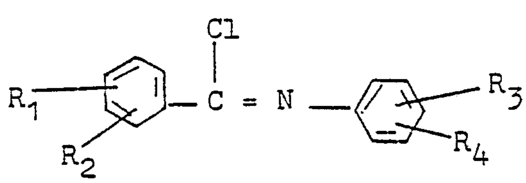

(III)

zu erhalten, das man der Einwirkung eines Hydrazids der Formel IV

$$H_2N—NH—CO—Z \qquad (IV)$$

in racemischer oder optisch aktiver Form unterzieht, worin Z eine Gruppe

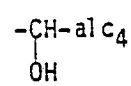

darstellt, in der $alc_4$ einen Alkylrest mit 1 bis 4 Kohlenstoffatomen wiedergibt, um eine Verbindung der Formel V in racemischer oder optisch aktiver Form zu erhalten,

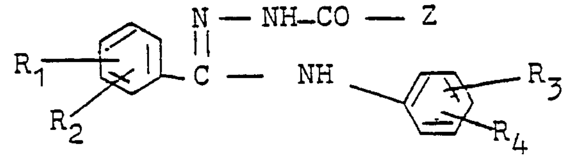

(V)

die man durch Erhitzen cyclisiert, um zu einer Verbindung der Formel ( I) in racemischer oder optisch aktiver Form zu gelangen, in der R eine Gruppe

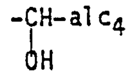

wiedergibt, die man gewünschtenfalls verestert, um ein Produkt der Formel (I) zu erhalten, in dem $R_A$ eine Gruppe

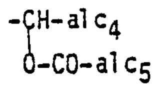

darstellt, und das Produkt der Formel (I) gewünschtenfalls der Einwirkung einer Säure unterzieht, um hieraus das Salz zu bilden.

2. Verfahren gemäß Anspruch 1, dadurch gekennzeichnet, daß man von einer Verbindung der Formel (II), worin einer der beiden Substituenten $R_1$ oder $R_2$ ein Wasserstoffatom und der andere Substituent entweder eine Methoxygruppe in para-Stellung oder eine Gruppe $NO_2$ oder eine Dimethylamino-Gruppe in para-Stellung bedeutet und worin einer der beiden Substituenten $R_3$ oder $R_4$ für ein Wasserstoffatom und der andere Substituent entweder für eine Methoxygruppe in para-Stellung oder eine Gruppe $NO_2$ oder eine Dimethylaminogruppe in para-Stellung steht, und einem Hydrazid der Formel IV ausgeht, worin Z für einen Rest —CHOH-$alc_4$ steht, in dem $alc_4$ einen Alkylrest mit 1 bis 4 Kohlenstoffatomen darstellt.

3. Verbindungen der Formel (IA)

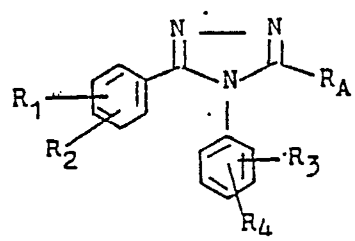

($I_A$)

worin $R_1$, $R_2$, $R_3$, $R_4$ und $R_A$ die in Anspruch 1 angegebenen Bedeutungen besitzen, in deren racemischen oder optisch aktiven Formen sowie deren Additionssalze mit Säuren.

4. Verbindungen der Formel (IA) gemäß Anspruch 3, worin einer der beiden Substituenten $R_1$ oder $R_2$ für ein Wasserstoffatom steht und der andere Substituent entweder eine Methoxygruppe in para-Stellung oder eine Dimethylaminogruppe in para-Stellung wiedergibt und worin einer der beiden Substituenten $R_3$ oder $R_4$ ein Wasserstoffatom und der andere Substituent entweder eine Methoxygruppe in para-Stellung oder eine Dimethylaminogruppe in para-Stellung bedeutet, in deren racemischen oder optisch aktiven Formen sowie deren Additionssalze mit Säuren.

5. 5-[4-(Dimethylamino)-phenyl]-(4-methoxyphenyl)-4-methyl-4H-1,2,4-triazol-3-methanol in racemischer oder optisch aktiver Form und dessen Additionssalze mit Säuren.

6. Als Arzneimittel die Verbindungen der Formel (IA) gemäß den Ansprüchen 3 und 4 sowie deren Additionssalze mit pharmazeutisch verträglichen Säuren.